# EUROPÄISCHE PATENTSCHRIFT

(11) **EP 1 962 887 B1**
(45) Veröffentlichungstag und Bekanntmachung des Hinweises auf die Patenterteilung: **19.08.2009**
(21) Anmeldenummer: 06804388.4
(22) Anmeldetag: 10.11.2006
(51) Int. Cl.: G01N 33/50

(54) **Verfahren zum Bestimmen der entzündungshemmenden Wirkung eines Peptids**
Process for determining the anti-inflammatory effect of a peptide
Procédé de détermination d'un effet anti-inflammatoire d'un peptide

(30) Priorität: 23.12.2005 AT 20672005
(43) Veröffentlichungstag der Anmeldung: 03.09.2008
(73) Patentinhaber: Fibrex Medical Research & Development GmbH, 1010 Wien (AT)
(72) Erfinder: PETZELBAUER, Peter, A-1230 Wien (AT); HENNING, Rainer, CH-8738 Uetliburg (CH)
(74) Vertreter: Schwarz, Albin
(86) Internationale Anmeldenummer: PCT/AT2006/000465
(87) Internationale Veröffentlichungsnummer: WO 2007/070899

(56) Entgegenhaltungen:
- WO-A-20/05086578
- WO-A-20/06000007
- PETZELBAUER P ET AL: "The fibrin-derived peptide Bbeta15-42 protects the myocardium against ischemia-reperfusion injury" NATURE MEDICINE, NATURE PUBLISHING GROUP, NEW YORK, NY, US, Bd. 11, Nr. 3, März 2005 (2005-03), Seiten 298-304, XP002350673 ISSN: 1078-8956
- LEDGERWOOD ANNA M ET AL: "A review of studies on the effects of hemorrhagic shock and resuscitation on the coagulation profile." JOURNAL OF TRAUMA INJURY INFECTION AND CRITICAL CARE, Bd. 54, Nr. 5 Supplement, Mai 2003 (2003-05), Seiten S68-S74, XP008077002 ISSN: 1079-6061
- LEE M E ET AL: "Fragment E derived from both fibrin and fibrinogen stimulates interleukin-6 production in rat peritoneal macrophages." MOLECULES AND CELLS 28 FEB 1999, Bd. 9, Nr. 1, 28. Februar 1999 (1999-02-28), Seiten 7-13, XP008081766 ISSN: 1016-8478
- STATON C A ET AL: "The role of fibrinogen and related fragments in tumour angiogenesis and metastasis" EXPERT OPINION ON BIOLOGICAL THERAPY 2003 UNITED KINGDOM, Bd. 3, Nr. 7, 2003, Seiten 1105-1120, XP009020273 ISSN: 1471-2598

## Beschreibung

### Hintergrund der Erfindung

Die vorliegende Offenbarung ist auf eine pharmazeutische Zusammensetzung zur Behandlung von hämorrhagischem Schock und seinen Folgeerscheinungen gerichtet.

Ein Schock ist eine akute Komplikation vieler verschiedener pathologischer Zustände, die durch die Unfähigkeit des Herz-Kreislauf-Systems, einen ausreichenden Durchblutungsdruck aufrechtzuerhalten, gekennzeichnet ist. Genauer gesagt übersteigt beim hämorrhagischen Schock der Blutverlust die Fähigkeit des Körpers, zu kompensieren und für eine ausreichende Gewebedurchblutung und Sauerstoffsättigung zu sorgen. Dies liegt häufig an einem Trauma, kann jedoch auch durch eine Spontanblutung (z.B. gastrointestinale Blutung, Entbindung), einen operativen Eingriff und andere Gründe verursacht sein.

Am häufigsten wird ein klinischer hämorrhagischer Schock durch ein akutes Blutungsgeschehen mit separatem Auslösungsvorgang verursacht. Weniger häufig ist ein hämorrhagischer Schock bei chronischen Zuständen mit subakutem Blutverlust zu beobachten. Dieser Zustand ist die Haupttodesursache in der Altersgruppe von 1-44.

Die physiologischen Kompensationsmechanismen bei einer Blutung umfassen eine anfängliche periphere und mesenterische Gefäßverengung, um Blut zum Zentralkreislauf zu verlagern. Dies wird dann durch eine fortschreitende Tachykardie verstärkt. Eine invasive Überwachung kann einen erhöhten Herzindex, eine gesteigerte Sauerstoffzufuhr (d.h. DO₂) und einen erhöhten Sauerstoffverbrauch (d.h. VO₂) durch die Gewebe offenbaren. Die Lactatspiegel, der Säure-Base-Zustand und andere Kennzeichen können ebenfalls nützliche Indikatoren für einen physiologischen Zustand sein. Das Alter, medikamentöse Behandlungen sowie Comorbid-Faktoren können die Reaktion eines Patienten auf einen hämorrhagischen Schock beeinflussen.

Ein Versagen der kompensierenden Mechanismen kann beim hämorrhagischen Schock zum Tod führen. Ohne Intervention ist bei einem schweren hämorrhagischen Schock eine klassische trimodale Verteilung der Todesfälle zu sehen. Innerhalb von Minuten nach einer Blutung tritt aufgrund einer sofortigen Ausblutung ein anfänglicher Mortalitätspeak auf. Ein weiterer Peak tritt aufgrund fortschreitender Dekompensation nach 1 bis mehreren Stunden auf. Ein dritter Peak tritt nach Tagen oder Wochen aufgrund einer Sepsis und eines Organversagens auf, die eine häufige Folgeerscheinung eines Reperfusionsschadens an den Organen sind.

Die Behandlung eines durch großen Blutverlust verursachten Schocks ist eine große Herausforderung, da sekundäre Wirkungen, die mit einer Entzündungsreaktion und Veränderungen des Gerinnungssystems einhergehen, möglicherweise unabhängig wurden und zum Tod des Patienten führen, ungeachtet der Frage, ob ein ausreichender Ersatz von Flüssigkeit und Blut möglich war. Eine spezifische Behandlung eines großen Blutverlusts infolge von Unfällen oder anderen Wunden und eines hämorrhagischen Schocks besteht in der Flüssigkeitsreanimation mit Kristalloiden oder Kolloiden zur Wiederherstellung der Organdurchblutung. Zusätzlich zielen derzeitige Verfahren darauf ab, die Symptome zu lindern, was eine mechanische Ventilation, den Ersatz von Flüssigkeit, die Anwendung herzwirksamer Arzneimittel, die strenge Kontrolle der Sauerstoffsättigung, des Hämoglobins, der Glucose und der Nierenfunktion einschließt. Die alleinige Kontrolle der Entzündungsreaktion, z.B. mittels hoch dosierter Steroide, oder die Hemmung der Gerinnung mit Antithrombin bringt keine Verbesserung der Überlebensrate.

Ein Schock infolge eines Blutverlusts und ein hämorrhagischer Schock hängen mit offensichtlichen oder nicht offensichtlichen Veränderungen des Plasmafibrinogens zusammen, begleitet von einer Fibrinbildung und einem Anstieg der Fibrinfragmente. Diese Aktivierung von Gerinnung und fibrinolytischen Pfaden kann zu einer offensichtlichen oder nicht offensichtlichen disseminierten intravaskulären Koagulation (DIC) führen, welche einen Gefäßverschluss und Endorganschaden zur Folge hat, und zu einem Verbrauch an Gerinnungsfaktoren, der Blutungen zur Folge hat. Wichtig ist, dass Fibrinogen, Fibrin und Fibrinfragmente nicht nur bei der Blutgerinnung eine Rolle spielen, sondern mehrere Bindungsstellen für Zell- und Matrixproteine aufweisen, welche ihnen das Wechselwirken mit weißen Blutkörperchen, Blutplättchen, Endothelzellen und Matrixstrukturen ermöglichen. Dies führt zur Zellaktivierung, Zellwanderung, einem Freisetzen von Cytokinen und letztendlich zu einer Entzündungsreaktion. Die Rolle, die Fibrinogen oder Fibrin bei der Entzündung spielt, ist umfassend dokumentiert (besprochen von Altieri Thromb Haemost 82:781-786; Herrick et al. Int J Biochem Cell Biol 31:741-46). Der D-Bereich des Moleküls enthält zahlreiche Bindungsstellen für Matrixmoleküle, Endothelzellen, Blutplättchen und Entzündungszellen. Der E-Bereich des Fibrins bindet an CD11c (Loike et al. Proc Natl Acad Sci USA 88:1044-48).

Vor kurzem beschrieben wir eine neue Rolle für die Bbeta₁₅₋₄₂-Sequenz des Fibrins bei der Entzündung (WO 02/48180). Diese Sequenz ist ebenfalls im E-Bereich des Fibrins lokalisiert und ist nur dann aktiv, wenn Fibrinopeptid gespalten wird. Fibrinfragmente, welche diese Sequenz an ihrem freien N-Terminus der beta-Kette enthalten, binden an das Endothelium und verursachen eine Entzündung, und ein Peptid, das mit den Aminosäuren 15-42 der Bbeta-Kette des Fibrins zusammenpasst, blockiert die Bindung von Fibrinfragmenten an die Endotheloberflächen und blockiert in vitro die Entzündung (WO 02/48180). In vivo verhindert dieses Peptid eine myokardiale Entzündung und verringert die Ausmaße eines Myocardinfarkts in Situationen der Ischämie / Reperfusion (WO 02/48180).

In WO2005/086578 wird ein Messverfahren zur Bestimmung der entzündungshemmenden Wirkung von Peptiden beschrieben, wobei eine Hemmung der T-Zellfunktion gemessen wird.

Fibrinfragmente treten in jedweder Situation mit beeinträchtiger Fibrinbildung und beeinträchtiger Fibrinolyse auf. Besonders in Situationen des Schocks stellen diese veränderte Fibrinbildung und diese veränderte Fibrinolyse ein großes Problem dar. Bei zahlreichen Erkrankungen wurde eine direkte Wechselbeziehung zwischen dem Ergebnis und der Beeinträchtigung der Fibrinbildung / Fibrinolyse dokumentiert. Z.B. Dengue (van Gorp et al. J Med Virol 2002, 67:549-54, Mairuhu et al. Lancet Inf Dis 2003; 3:33-41). Das Atemnotsyndrom beim Erwachsenen (ARDS) ist eine Form einer akuten Lungenschädigung, die durch eine voll ausgeprägte extravaskuläre Fibrinablagerung gekennzeichnet ist (Idell Am J Respir Med. 2002; 1:383-91). Eine Thrombose in den pulmonalen Gefäßen und eine disseminierte intravaskuläre Koagulation wurden im Zusammenhang mit ARDS ebenfalls beobachtet.

### Kurzfassung der Erfindung

Die Offenbarung betrifft die Verwendung eines Peptids, umfassend die N-terminale Sequenz 
Gly-His-Arg-Pro-Leu-Asp-Lys-Lys-Arg-Glu-Glu-Ala-Pro-Ser-Leu-Arg-Pro-Ala-
-Pro-Pro-Pro-Ile-Ser-Gly-Gly-Gly-Tyr-Arg
 oder irgendeiner allelen Variante oder Derivats dieses Peptids, welche bzw. welches die biologische Eigenschaft besitzt, mit dem induzierbaren VE-Cadherin-Bindungsmotiv an der Bβ-Kette (d.h. Bβ₁₅₋₄₂) des menschlichen Fibrins zusammenzupassen, für die Herstellung einer pharmazeutischen Zusammensetzung zur Behandlung von Schock.

Bei einer bevorzugten Ausführungsform ist dieses Peptid

Gly-His-Arg-Pro-Leu-Asp-Lys-Lys-Arg-Glu-Glu-Ala-Pro-Ser-Leu-Arg-Pro-Ala-
-Pro-Pro-Pro-Ile-Ser-Gly-Gly-Gly-Tyr-Arg

Wir stellten überraschenderweise fest, dass diese Peptide beim Schutz warmblütiger Tiere vor den Folgen und Komplikationen einer Reperfusion nach größeren Blutungsvorfällen und eines hämorrhagischen Schocks eine äußerst günstige Wirkung aufweisen. Wir konnten zeigen, dass sie in der Lage sind, den Organschaden und das Organversagen zu verhindern, welche auf akute, durch hämorrhagischen Schock und Reperfusion verursachte Entzündungsreaktionen zurückzuführen sind. Die Peptide können solche Erkrankungen wie das systemische inflammatorische Response-Syndrom (SIRS), das akute Atemnotsyndrom (ARDS), Nierenversagen, Leberversagen und Multiorganversagen verhindern.

### Detaillierte Beschreibung der Erfindung

### Peptide und Proteine

Peptide wurden durch eine Festphasen-Peptidsynthese hergestellt und mittels einer Umkehrphasen-HPLC gereinigt, wobei Nucleosil 100-10C18-Säulen (PiChem, Graz, Österreich) verwendet wurden. Es sollte beachtet werden, dass der beta 15-42-Bereich unter Spezien 100% gleichartig ist, wenn konservative Aminosäure-Substitutionen zugelassen werden. Der aus den Aminosäuren Aα1-51, Bβ1-118 und γ1-78 zusammengesetzte N-terminale Disulfidknoten von Fibrinogen (NDSK) wurde wie zuvor beschrieben hergestellt (WO 02/48180). Der aus den Aminosäuren Aα17-51, Bβ15-118 und γ1-78 zusammengesetzte N-terminale Disulfidknoten von Fibrin (NDSK-II, dem die Fibrinopeptide A und B fehlen) wurde hergestellt, indem NDSK bei 37 °C 3 Stunden lang mit Thrombin (20 U / 1mg NDSK) behandelt wurde. Das restliche Thrombin wurde bei 37 °C 2 Stunden lang mit 10 mM Diisopropylfluorophosphat (Fluka, Milwaukee, WI) neutralisiert. Alle Produkte wurden daraufhin in eine phosphatgepufferte Salzlösung (PBS) dialysiert.

### ELISA

### Peptid Bβ₁₅₋₄₂ bindet an VE-Cadherin

Die Wechselwirkung der Bbeta-Kette (Bbeta₁₅₋₄₂) von Fibrin mit Endothelzellen verursacht morphologische Veränderungen (Bunce et al. J Clin Invest 89:842-50; Bach et al. Exp Cell Res 238:324-34; Chalupowicz et al. J Cell Biol 130:207-15; Hamaguchi et al. Blood 81:2348-56; Francis et al. Blood cells 19:291-306), eine Proliferation (Sporn et al. Blood 86:1802-10), das Freisetzen von von-Willebrand-Faktor (Ribes et al. J Clin Invest 79:117-23, Ribes et al. J Clin Invest 84: 435-42; Erban und Wagner, J Biol Chem 267, 2451-58) und möglicherweise IL-8 (Qi et al. Blood 90:3593-3602) und eine Membranexpression von CD54 (Harley et al. Art Thromb Vasc Biol 20:652-658). VE-Cadherin wurde als Bindungsligand der Sequenz Bbeta₁₅₋₄₂ identifiziert und ELISAs wurden entwickelt, um diese Wechselwirkung von Endothelzellen und/oder VE-Cadherin mit Fibrin oder Fibrinfragmenten nachzuweisen. Martinez et al. verwendeten anti-Pan-Cadherin-Antikörper zum Einfangen von Cadherinen aus Endothelzellen, gefolgt von einer Inkubation mit Fibrin (Martinez et al. Ann NY Acad Sci 936:386-405), HUVEC-Monoschichten (welche VE-Cadherin exprimieren) wurden mit radiomarkierten Fibrinfragmenten oder Peptid Bbeta₁₅₋₄₂ überschichtet (Bach et al. J Biol Chem 273:30719-28; Harley et al. Art Thromb Vasc Biol 20:652-658), und von Gorlatov und Medved wurde rekombinantes VE-Cadherin verwendet (Biochemistry 41:4107-16). Andere setzten zum Nachweisen von Fibrinfragmenten im Blut einen ELISA ein, wobei sie hauptsächlich Antikörper gegen verschiedene Sequenzen innerhalb des Fibrinogenmoleküls verwendeten, einschließlich Antikörpern gegen das Bbeta₁₅₋₄₂-Motiv (besprochen bei Fareed et al. Clin Chem 8:1845-53).

Wir entwickelten einen modifizierten ELISA, der mit denselben, von anderen beschriebenen Prinzipien arbeitet, der Zweck des hier beschriebenen ELISA besteht jedoch nicht darin, Fibrin-Abbauprodukte quantitativ zu bestimmen, sondern nach Proteinen, Peptiden oder Verbindungen zu suchen, welche die Bindung der Bbeta₁₅₋₄₂-Sequenz und des VE-Cadherins stören. Das Prinzip besteht darin, dass das VE-Cadherin entweder als verkürztes Protein, als Vollprotein oder gekoppelt mit anderen Proteinen, welche die Bbeta₁₅₋₄₂-Bindungsstelle nicht stören, mit der Bbeta₁₅₋₄₂-Sequenz von Fibrin wechselwirken darf. Man kann jegliche andere zusätzliche Substanz in dieses System einbringen und messen, ob diese Substanz die VE-Cadherin/Bbeta₁₅₋₄₂-Bindung hemmt.

Im Einzelnen wurden 96-Mulden-Proteinimmobilisierungsplatten (Exiqon, Vedbaek, DK) mit rekombinantem menschlichem VE-Cadherin-FC-Fusionsprotein (8 nM/ml; R&D Systems, Minneapolis) in PBS beschichtet und über Nacht bei 4 °C stehengelassen. Die Platten wurden danach gewaschen und mit Peptid Bβ₁₅₋₄₂ (GHRPLDKKREEAPSL RPAPPPISGGGYR), das am C-Terminus des Peptids mit einer FLAG-Sequenz (DYKDDDDK) markiert war, oder mit einem FLAG-markierten Zufallspeptid (DRGAPAHRPPRGPISGRSTPEKEKLLPG) inkubiert, und zwar in einer Konzentration von 0-80 µMol/ml. Nach dem Waschen wurde durch Inkubation mit einem Peroxidasemarkierten anti-FLAG-Antikörper (Sigma, St. Louis, USA) und einem chromogenen Substrat gebundenes FLAG-markiertes Peptid nachgewiesen. Die optische Dichte wurde durch einen auf eine Wellenlänge von 450 nm eingestellten ELISA-Plattenleser bestimmt. Die Daten stellen den Mittelwert aus drei unabhängigen Versuchen dar, wobei jeder dreifach durchgeführt wurde. Die untenstehende Tabelle zeigt, dass das Peptid Bβ₁₅₋₄₂ in konzentrationsabhängiger Weise an VE-Cadherin band. Im Gegensatz dazu zeigte das Zufallspeptid nur eine unwesentliche Bindung.

**Dosisabhängige Bindung von Peptid Bbeta₁₅₋₄₂ an VE-Cadherin**

| | | | | | | | | | | | |
|---|---|---|---|---|---|---|---|---|---|---|---|
| µM/ml | | 0 | 0,23 | 0,7 | 2,3 | 7 | 14 | 21 | 35 | 46 | 70 |
| 15-42 FLAG | Mittelwert | 0 | 0,01 | 0,02 | 0,08 | 0,33 | 0,92 | 1,3 | 1,5 | 1,93 | 2,1 |
| | Standard abweichung | 0 | 0,01 | 0,01 | 0,03 | 0,17 | 0,19 | 0,2 | 0 | 0 | 0 |
| | | | | | | | | | | | |
| Zufalls FLAG | Mittelwert | 0 | 0,01 | 0 | 0,01 | 0,03 | 0,12 | 0,2 | | 0,35 | 0,5 |
| | Standard abweichung | 0 | 0,01 | 0 | 0,01 | 0;02 | 0,04 | 0,1 | | 0 | 0 |

Das Peptid Bβ₁₅₋₄₂ und Fibrinfragmente konkurrieren hinsichtlich der Bindung an VE-Cadherin.

In einem nächsten Schritt analysierten wir, ob dieser ELISA zum Screenen nach anderen Peptiden/Verbindungen verwendet werden kann, die mit der Bindung der Bβ₁₅₋₄₂-Sequenz an VE-Cadherin konkurrieren. Erwartungsgemäß hemmte das Peptid Bβ₁₅₋₄₂ vollständig die Bindung des flag-markierten Peptids Bβ₁₅₋₄₂ und wurde als positive Kontrolle verwendet, und Zufallspeptide oder Lösungsmittel hatten keinerlei Wirkung und wurden als negative Kontrollen verwendet. Kürzere Peptide hemmten teilweise die Bindung von Bβ₁₅₋₄₂ an VE-Cadherin. NDSK-II hemmte die Bβ₁₅₋₄₂-Bindung in konzentrationsabhängiger Weise. Ein Gleichgewicht zwischen Bβ₁₅₋₄₂ und NDSK-II (50%ige Hemmung) war bei einem Molverhältnis von 24:1 erreicht. NDSK hatte wenig oder keine Winkung.

Die Kunststoffoberfläche wurde mit VE-Cadherin in einer Konzentration von 8 nM/ml beschichtet. Danach wurden die angegebenen Peptide in Konzentrationen von 200 µM/ml hinzugefügt, NDSK oder NDSK-II wurde in den angegebenen Konzentrationen hinzugefügt. Der Nachweis der Bindung des FLAG-markierten Bbeta₁₅₋₄₂ (I2 µM/ml) wurde wie obenstehend beschrieben durchgeführt.

Außerdem identifizierten wir eine spezifische Analyse zum Bestimmen der behaupteten Aktivität der Peptide, welche in einer Messung der Hemmung des Freisetzens von

Interleukin-6 aus Endothelzellen besteht. Interleukin-6 erwies sich als starkes Mittel zum Vorhersagen des Ausgangs und der Sterblichkeit in der Situation eines Schocks und ist daher ein wertvoller Parameter zum Bestimmen der günstigen Wirkungen der Peptide.

Die Erfindung berifft daher ein Verfahren zum Bestimmen der entzündungshemmenden Wirkung eines Peptids durch Messung der Hemmung des Freisetzens von Interleukin-6 (IL-6) aus Endothelzellen nach einer Exposition an das Fibrin E-Fragment.

| Blockierendes Reagens | % Hemmung der 15-42FLAG-Bindung an VE-Cadherin |
|---|---|
| | Mittelwert ± Standardabweichung |
| Peptid 15-42 (28mer) | 100 ± 10 |
| Peptid zufällig (4mer) | 3 ± 3 |
| Peptid zufällig (28mer) | 10 ± 3 |
| Lösungsmittel | 0 + 0 |
| Peptid 15-18 (4mer) 200 µM/ml | 65 ± 12 |
| Peptid 15-26 (12mer) 200 µM/ml | 64 ± 10 |
| Peptid 15-30 (16mer) 200 µM/ml | 61 ± 13 |
| Peptid 15-34 (20mer) 200 µM/ml | 67 ± 17 |
| Peptid 15-37 (24mer) 200 µM/ml | 17 ±19 |
| Peptid 16-42 (27mer) 200 µM/ml | 55 ±13 |
| | |
| Peptid 15-18 (4mer) 12 µM/ml | 7 ± 2 |
| Peptid 15-26 (12mer) 12 µM/ml | 6 ± 1 |
| Peptid 15-30 (16mer) 12 µM/ml | 6 ± 3 |
| Peptid 15-34 (20mer) 12 µM/ml | 7 ± 1 |
| Peptid 15-37 (24mer) 12 µM/ml | 7 ± 2 |
| Peptid 16-42 (27mer) 12 µM/ml | 5 ± 2 |
| | |
| NDSK-II 0,06 µM/ml | 1 + 0 |
| NDSK-II 0,12 µM/ml | 39 + 18 |
| NDSK-II 0,20 µM/ml | 42 + 14 |
| NDSK-II 0,60 µM/ml | 52 + 16 |
| NDSK-II 1,2 µM/ml | 63 + 13 |
| NDSK-II 2,4 µM/ml | 79 + 9 |
| NDSK-II 4,0 µM/ml | 82 + 12 |
| NDSK 0,06 µM/ml | 0 + 0 |
| NDSK 0,12 µM/ml | 2 + 1 |
| NDSK 0,20 µM/ml | 1 + 1 |
| NDSK 0,60 µM/ml | 7 + 6 |
| NDSK 1,2 µM/ml | 15 + 13 |
| NDSK 2,4 µM/ml | 16 + 9 |
| NDSK 4,0 µM/ml | 20 + 10 |
| anti-VE-Cadherin Ab (TEA1/31, 1 mg/ml) | 2 + 1 |

### Hemmung des Freisetzens von Interleukin-6 aus Endothelzellen

96-Mulden-Zellkulturplatten wurden mit 1%iger Gelatine beschichtet. In jede Mulde wurden etwa 23.000 menschliche Nabelschnurvenenendothelzellen (HUVEC) in 200 µl Medium pipettiert. Die HUVECs wurden der Passage 4 aus einem Pool von vier verschiedenen Donoren entnommen. Das für die Verdünnung verwendete Medium war ein Standardmedium mit 10% FCS, ECGS in *Iscove's modified Dulbecco medium* (Petzelbauer et al., J Immunol. 151:5062-5072, 1993). Innerhalb von ungefähr 18 Stunden bildet sich bei 37°C in einem Inkubator eine konfluente Zellmonoschicht. Der Überstand wurde entfernt und durch ein Medium ersetzt, das entweder NDSK-II allein oder NDSK-II plus Peptid Bβ₁₅₋₄₂ (GHRPLDKKREEAPSLRPAPPPISGGGYR) enthielt.
Das endgültige Gesamtvolumen betrug 200 µl. Nach einer 4-stündigen Inkubation bei 37°C unter 5%igem CO₂ wurde der Zellkulturüberstand erneut entfernt und 10 Minuten lang bei 4°C mit 3000 U/min zentrifugiert. Die Konzentration an Interleukin-6 (IL-6) wurde unter Anwendung einer handelsüblichen Analyse gemessen (IL-6 Quantikine Immunoassay (R&D Sytems; Catalog#: D6050; Lot: 231361). Die Kalibrierung wurde gemäß dem vom Hersteller der Analyse bereitgestellten Standardprotokoll mit verschiedenen Verdünnungen von rekombinantem IL-6 und Medium durchgeführt. Die Ablesungen der optischen Dichte erfolgten bei einer Wellenlänge von 450 nm mit einer Referenzwellenlänge von 550 nm.

**Ergebnisse**

| | IL-6-Konzentration in pg/ml |
|---|---|
| | |
| Medium | 12±1 |
| 0,3 µg/ml NDSK-II | 19±2 |
| 0,3 µg/ml NDSK-II + 10 µg/ml Peptid | 15±2 |
| 3 µg/ml NDSK-II | 25±3 |
| 3 µg/ml NDSK-II + 10 µg/ml Peptid | 12±3(p<0,05 vergl. |
| | mit NDSK-II allein) |
| 30 µg/ml NDSK-II | 21±2 |
| 30 µg/ml NDSK-II + 10 µg/ml Peptid | |
| | 13±1(p<0,05 vergl. |
| | mit NDSK-II allein) |

### Hämorrhagischer Schock bei Hausschweinen

Männliche Landrace-Hausschweine mit einem Gewicht von 20-35 kg wurden mit Standardkost gefüttert und erhielten Wasser ad libitum. Alle Vorgänge wurden in Übereinstimmung mit den AAALAC-Richtlinien und gemäß dem *Handbuch für die Pflege von Labortieren* (Amt für Gesundheit und Soziales, Nationale Gesundheitsinstitute, Veröffentlichung Nr. 86-23) ausgeführt. Die Schweine wurden mit Azaperon (4 mg/kg) medikamentös vorbehandelt. In die Ohrvene wurde eine Kanüle eingeführt, und das Anästhetikum Propofol wurde verabreicht. Die Tiere wurden 1 Stunde lang vollständig instrumentiert und äquilibriert, um eine stabile Basislinie zu erhalten. Anschließend wurden die Tiere (n=16) ausgeblutet, bis ein mittlerer Arteriendruck von 40 mm Hg erreicht wurde. Sie wurden 1 Stunde lang bei diesem Pegel, der einen hämorrhagischen Schock darstellte, gehalten. Nach diesem Zeitraum wurde das Volumen des verlorenen Blutes durch das vergossene Blut und Salzlösungen (Kristalloide) ersetzt. Außerdem erhielten die Tiere eine Bolusdosierung des Peptids Bβ15-42 (n=8) oder ein Peptid mit Zufallssequenz (n=8) in einer Dosierung von 2,4 mh/kg, und zwar jeweils als einzelnen intravenösen Bolus. Während der anschließenden Reperfusionsperiode von 5-stündiger Dauer wurden der Horrowitz-Index (Messung der Sauerstoffsättigung des Blutes), der PEEP (positiver endexpiratorischer Druck in mm Hg) und das Herzausstoßvolumen zu verschiedenen Zeitpunkten bestimmt.

In den nachfolgenden Tabellen wurden die folgenden Zeitpunkte verwendet:
Zeitpunkt 1: 1 Stunde vor dem hämorrhagischen Schock
Zeitpunkt 2: während des hämorrhagischen Schocks
Zeitpunkt 3: 1 Stunde nach Beginn der Reperfusion
Zeitpunkt 4: 3 Stunden nach Beginn der Reperfusion
Zeitpunkt 5: 5 Stunden nach Beginn der Reperfusion

| EV (ml) | | | |
|---|---|---|---|
| Kontrollgruppe | | behandelte Gruppe | |
| 1: | 3 8 +/- 1 | 44 +/- 2 | |
| 2: | 6 +/- 1 | 6 +/- 1 | |
| 3: | 25 +/- 3 | 31 +/- 4 | p<0,05 |
| 4: | 25 +/- 4 | 32 +/- 3 | p<0,05 |
| 5: | 22 +/- 2 | 37 +/- 4 | p<0,05 |

| Horrowitz-Index | | | |
|---|---|---|---|
| Kontrollgruppe | | behandelte Gruppe | |
| 1: | 460 +/- 20 | 465 +/- 20 | |
| 2: | 395 +/- 60 | 450 +/- 20 | |
| 3: | 425 /- 30 | 420 +/- 10 | |
| 4: | 360 +/- 50 | 410 +/- 20 | |
| 5: | 220 +/- 20 | 420 /- 30 | p<0.05 |

| PEEP (mmHg) | | | |
|---|---|---|---|
| Kontrollgruppe | | behandelte Gruppe | |
| 1: | 4+/-0,2 | 4 +/- 0,1 | |
| 2: | 4,3 /- 0,3 | 4 /- 0,1 | |
| 3: | 5 +/- 0,3 | 4,1 +/- 0,2 | p<0,05 |
| 4: | 5,4 +/- 0,4 | 4,2 +/- 0,3 | p<0,05 |
| 5: | 6,6 +/- 0,4 | 4,3 +/- 0,2 | p<0,05 |

## Patentansprüche

1. Verfahren zum Bestimmen der entzündungshemmenden Wirkungen eines Peptids durch Messung der Hemmung des Freisetzens von Interleukin-6 (IL-6) aus Endothelzellen nach einer Exposition an das Fibrin E-Fragment.

## Claims

1. A method of determining the anti-inflammatory effects of a peptide by measuring the inhibition of release of interleukin-6 (IL-6) from endothelial cells after a challenge with fibrin E fragment.

## Revendications

1. Procédé de détermination de l'effet anti-inflammatoire d'un peptide par mesure de l'inhibition de la libération de l'interleukine-6 (IL-6) issue de cellules endothéliales après exposition au fragment E de la fibrine.
